# EUROPEAN PATENT SPECIFICATION

(11) **EP 2 185 110 B1**
(45) Date of publication and mention of the grant of the patent: **06.07.2016**
(21) Application number: 08798686.5
(22) Date of filing: 26.08.2008
(51) Int. Cl.: A61F 5/44

(54) **FLUID FILLED SEAL FOR CONTACTING THE HUMAN BODY**
FLÜSSIGKEITSGEFÜLLTE ABDICHTUNG FÜR KONTAKT MIT DEM MENSCHLICHEN KÖRPER
JOINT REMPLI DE FLUIDE POUR LA MISE EN CONTACT AVEC LE CORPS HUMAIN

(30) Priority: 27.08.2007 US 968099 P
(43) Date of publication of application: 19.05.2010
(73) Proprietor: ConvaTec Technologies Inc., Las Vegas, NV 89169-6754 (US)
(72) Inventor: CLINE, John, Skillman, NJ 08858 (US)
(74) Representative: Mays, Julie
(86) International application number: PCT/US2008/074295
(87) International publication number: WO 2009/029610

(56) References cited:
- EP-A1- 1 346 711
- EP-A1- 1 348 412
- EP-A2- 1 795 157
- WO-A1-90/07311
- US-A- 6 033 390
- US-A1- 2006 206 069
- US-A1- 2007 123 832
- US-A1- 2007 191 794

## Description

### FIELD OF THE INVENTION

The present invention relates to a controlled evacuation ostomy appliance having a fluid filled seal for contacting the human body, for forming a seal at, near or around, a body orifice. The seal is especially suitable for use in an ostomy appliance, and may also be utilized in an anal fecal incontinence device and a catheter.

### BACKGROUND TO THE INVENTION

U.S. Patent No. 6,723,079 and EP-A-1348412 describe controlled ostomy evacuation devices including an inflatable membrane seal, consisting of a membrane at least partly enclosing a fluid filled inflation chamber. The membrane seal is intended to create a temporary conformal closure of the stoma. When the device is placed over the stoma and the inflation chamber is inflated, the membrane seal conforms to and bears against the stoma with a distributed contact force that is dependent on the pressure of inflation fluid in the chamber. The membrane seal blocks release of stool from the stoma while, at the same time, being intended to allow venting of flatus by separating locally a small distance from the stoma tissue under the pressure of the escaping flatus.

The inflatable volume of the prior art seal is closed by means of a check valve that permits injection of inflation fluid, such as air or saline, from a pump or syringe. The check valve prevents discharge of inflation fluid from the chamber, since this results in irrecoverable loss of inflation pressure and consequent loss of contact pressure against the stoma, resulting in risk accidental leakage of stool past the seal.

One embodiment of EP-A-1348412 includes a resilient foam support positioned behind and outside the fluid filled chamber. The foam support provides a spring action behind the inflatable volume, without changing the inflation characteristics of the fluid filled chamber. The spring action can partly compensate for accidental partial loss of inflation fluid from the closed inflation volume, or partly accommodate distance changes between the stoma and the cap of the device.

In devising the present invention, the inventor has appreciated new issues that would be desirable to address. It is important that, when the membrane seal is in contact with the stoma, contact pressure between the stoma and the membrane seal is kept in a narrow range, namely, as low as possible (to ensure good blood perfusion in the stoma tissue) while maintaining an effective temporary seal against discharge of stool. However, it is difficult to keep the contact pressure in such a narrow range because, for a given amount of inflation fluid in the chamber, any change in chamber volume caused by movement of the stoma, directly affects the inflation pressure.

The inventor has further appreciated that, during the wear time of an ostomy appliance, the stoma can move dynamically inwardly towards the body and/or outwardly away from the surface of the peristomal skin over a total distance that can exceed 1 cm. This movement can be due to peristaltic motion of the bowel, impending release of stool or gas from the stoma, or muscular contractions of the abdomen. Under conditions when the stoma moves inwardly towards the body (i.e., increasing the volume of the inflation chamber), the contact pressure between the membrane seal and the stoma can fall, increasing the risk of leakage of stool if the contact pressure is too low. In contrast, under conditions when the stoma or its contents pushes outwardly against the membrane seal (reducing the volume of the inflation chamber), contact pressure between the seal and the stoma can potentially rise. During such times, the increased contact pressure may result in undesirable reduced blood perfusion in the stoma. The duration of such conditions may be highly unpredictable, some lasting only seconds, others minutes, and sometimes several hours.

The present invention has been devised having appreciated the above issues.

### SUMMARY OF THE INVENTION

The invention is defined in claim 1. Preferred embodiments are defined in dependent claims 2-14. The invention provides a controlled evacuation ostomy appliance having a fluid filled seal, the seal comprising: a fluid impermeable membrane that forms a movable wall of a (first) fluid chamber; one or more ports communicating with the chamber; and a resilient device disposed within the fluid chamber. The resilient device may be configured to urge the membrane in a direction (i) for forming a seal in use and/or (ii) for expanding the chamber.

With such a configuration, the degree of distention of the chamber is a function of both the fluid volume within the chamber, and the resilient device also within the chamber. This can enable the chamber volume to be managed dynamically to accommodate changes in the degree of stoma protrusion. Should the stoma move inwardly, the resilient device can apply a force to expand the chamber to compensate for stoma movement. This contrasts with the foam arrangement described above in the prior art, in which a foam spring behind the inflation chamber always tends to compress the chamber from behind, and can never expand the chamber as a way of compensating for stoma movement.

The sealing pressure exerted by the membrane is a function of the resilient force exerted by the resilient device and the pressure of fluid in the chamber. The port is configured to control the admission and/or discharge of fluid with respect to the chamber. The characteristics of the port determine how the seal adapts in response to an increase or decrease in the inflation pressure. For example, if the inflation pressure falls below a certain threshold (in the case that the stoma moves inwardly, and the chamber volume increases), the port is configured to allow entry of additional inflation fluid to restore the inflation pressure. Should the inflation pressure increase (in the case that the stoma moves outwardly, and the chamber volume decreases), the port may be configured to obstruct, or at least slow, exhaust of fluid from the chamber. This may enable the seal to withstand a short-term challenge from the stoma, but without maintaining a high contact pressure for an extended time period, since the fluid can escape over time. The port may control a damping action for the contraction/expansion of the resilient device. The damping action may be different in the compression direction from the expansion direction.

Other aspects of the invention are summarized by one or more features, or any combination of features discussed further below.

The present invention is a controlled evacuation ostomy appliance, wherein the appliance contains a membrane seal, and wherein the membrane seal contains resilient foam. Acceptable foam includes an open cell foam or a closed cell foam. The shape of foam may be a cylinder with a circular cross-section; a block with an elliptical cross section; or a block with a polygonal cross section. A surface of the foam facing towards or away from the stoma includes shapes selected from concave with a conical profile or a profile formed by a swept circular arc; convex with a conical profile or a profile formed by a swept circular arc.

A surface of the foam facing the stoma could be a smooth "skinned" surface. One or more of the surfaces of the foam may have a random texture, such as ridge or groove in a geometric pattern, e.g., a polygon; a series of radial rays; one or more circumferential rings. The pattern may be a repeating one.

The membrane seal desirably incorporates one or more openings to allow fluid to enter and exit the membrane seal in response to forces applied to the seal. The one or more openings incorporate a fluid flow restriction. The restriction may be provided by a small hole; a portion of a microporous membrane; a section of perforated film; or a porous plug.

Also, the membrane seal preferably incorporates an inlet check valve and an exhaust valve that opens when a predetermined pressure is exceeded. The exhaust valve remains permanently open once it has been opened. The fluid exhausts through a seal that is ruptured or broken once the predetermined pressure has been exceeded.

The present invention can also be described as a controlled evacuation ostomy appliance containing a membrane seal, wherein fluid enters the membrane seal through a closeable first valve, and wherein fluid exhausts from the membrane seal through a second opening.

The second opening is a valve that opens when a predetermined pressure is exceeded. The valve remains permanently open once it has been opened. Fluid exhausts through a seal that is ruptured or broken once the predetermined pressure has been exceeded.

The second opening may incorporate a fluid flow restriction (e.g. compared to the first opening when open). The restriction is a small hole; a portion of microporous membrane; a portion of perforated film; or a porous plug.

The membrane seal contains resilient foam. The first valve is an inlet check valve. The first valve incorporates a fluid flow restriction. The membrane seal is connected to a second volume. The passage of fluid from the membrane seal into the second volume is restricted. The passage of fluid from the second volume into the membrane seal is restricted. The passage of fluid from the membrane seal into the second volume opens when a predetermined pressure is exceeded. The passage of fluid from the second volume into the membrane seal opens when a predetermined pressure is exceeded.

While certain features have been identified above and in the appended claims, protection may be sought for any patentable feature described herein and/or illustrated in the drawings, whether or not emphasis has been placed thereon.

### BRIEF DESCRIPTION OF THE DRAWINGS

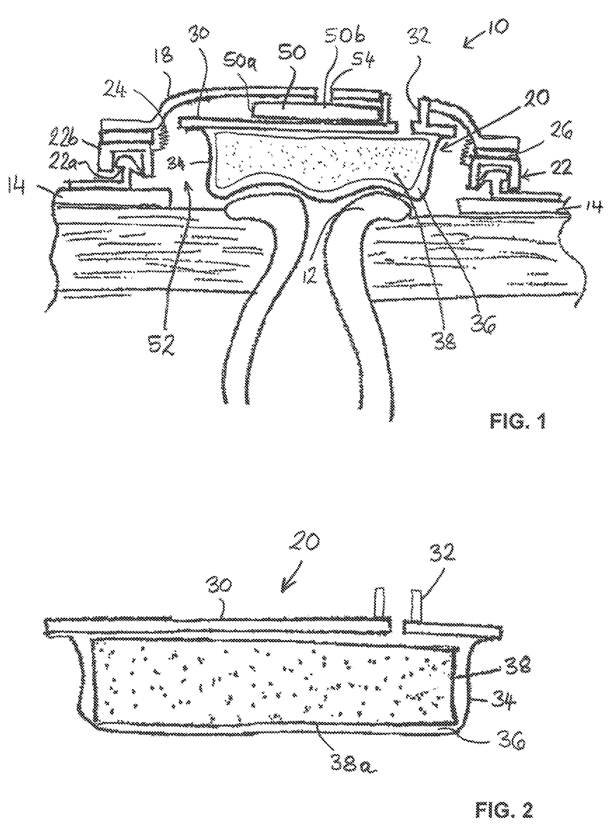

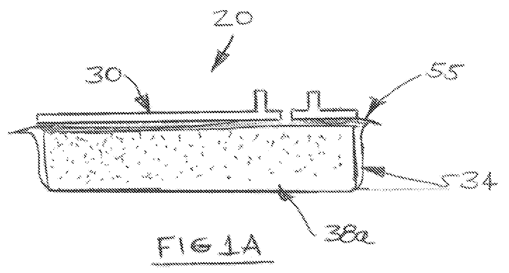

Fig. 1 is a schematic sectional view through a first embodiment of controlled discharge ostomy appliance with a fluid filled seal.
Fig. 1 a is a schematic sectional view showing, in more detail, a first embodiment of the fluid filled seal sub-assembly for the appliance of Fig. 1.
Fig. 2 is a schematic sectional view showing, in more detail, a second embodiment of the fluid filled seal sub-assembly for the appliance of Fig. 1.
Fig. 3 is an underside perspective view of a foam piece resilient member of the sub-assembly of Fig. 2.
Fig. 4 is a schematic sectional view through the seal sub-assembly of a second embodiment.
Fig. 5 is a schematic sectional view through the seal sub-assembly of a third embodiment.
Fig. 6 is a schematic perspective view showing a flap valve.
Fig. 7 is a schematic perspective view showing a duck-bill valve.
Fig. 8 is a schematic sectional view showing an umbrella valve;
Fig. 9 is a schematic perspective view showing a ball valve.
Fig. 10 is a schematic perspective view showing a poppet valve.
Fig. 11 is an exploded schematic perspective view showing a microporous port.
Fig. 12 is a schematic perspective view of a porous plug port.
Fig. 13 is a schematic perspective view of a leaky flap valve.
Fig. 14 is a schematic sectional view showing a rupture safety valve prior to rupture.
Fig. 15 is a schematic sectional view similar to Fig. 14, but showing rupture of the valve.
Fig. 16 is a schematic sectional view through the seal sub-assembly of a fourth embodiment, showing expansion of the seal.
Fig. 17 is a schematic sectional view similar to Fig. 16, but showing compression of the seal.
Fig. 18 is a schematic perspective view of the seal sub-assembly of Figs. 16 and 17.

### DETAILED DESCRIPTION OF PREFERRED EMBODIMENTS

The preferred embodiments of the invention are now described with reference to the accompanying drawings. The same reference numerals are used where appropriate to indicate the same or similar features.

Referring to Fig. 1, a controlled evacuation or discharge ostomy appliance device is illustrated employing a fluid filled seal for contacting the human body at, near or around an opening or orifice in the body, to form a seal against body tissue. The present embodiment, the medical device is an ostomy appliance 10 for a stoma 12, but the invention is applicable to incontinence management devices and catheters.

The ostomy appliance 10 generally comprises an apertured adhesive body fitment 14 for adhesive attachment to peristomal skin 16, a housing 18 supported by the apertured adhesive body fitment 14, and a fluid filled seal 20 mounted in or on the housing 18 for forming a seal with respect to the stoma 12. The apertured adhesive body fitment 14 includes a skin-friendly medical grade adhesive, such as a hydrocolloid containing adhesive. The housing 18 may be integral with the apertured adhesive body fitment 14, or it may be releasably coupled to the apertured adhesive body fitment by means of a coupling 22. In the present embodiment, the coupling 22 comprises interengageable mechanical coupling rings 22a and 22b. Also, in the present embodiment, an optional body waste collector 24 is provided in the housing 18. The body waste collector 24 is made of flexible plastics film. The body waste collector 24 may also be tubular, or it may be fabricated from one or more sheets of film. In Fig. 1, the body waste collector 24 is shown in its collapsed condition, and is mounted between the coupling 22 and the housing 18. The body waste collector 24 is kept in its initially compact state by means of a second releasable coupling connection (illustrated schematically at 26) between the housing 18 and the coupling ring 22b.

The fluid filled seal 20 generally comprises a support wall 30 having at least one port 32 defined therein, and a flexible membrane 34 depending from the support wall 30. The flexible membrane 34 and the support wall 30 together define and substantially enclose a chamber 36. The chamber 36 is substantially closed, except for the port 32. The flexible membrane 34 is made of generally flexible plastics film, and is impermeable to inflation fluid. In the present embodiment, the fluid is air but, as explained later, other gases or liquids may be used as desired. The flexible membrane 34 acts as a movable wall of the chamber 36. In the present embodiment, the flexible membrane 34 provides the seal surface for contacting the tissue of the stoma 12. The support wall 30 is made of plastics generally stiffer than the flexible membrane 34, to provide a self-supporting shape. The flexible membrane 34 is sealed to the support wall 30, for example, by a weld or by an adhesive bond.

In an alternative embodiment, see Fig. 1a, the flexible membrane 34 may depend from a separate component 55, that is, in turn, depended from the support wall 30. The separate component may, for example, be a plastic film component that incorporates a valve, port, or other functional feature.

A resilient device 38 is provided in the chamber 36 to urge the flexible membrane 34 into an expanded shape, distanced from the support wall 30 and/or to urge the flexible membrane 34 towards a sealing position with respect to the stoma 12. In the present embodiment, the resilient device 38 comprises resilient foam. The resilient device 38 is dimensioned so as to be a generally tight fit in the chamber 36, thereby resiliently holding the flexible membrane 34 in the expanded shape. For example, the foam 38 could have a natural shape larger than the chamber 36 size, so that the foam 38 is permanently in a state of at least partial compression. The shape of the foam 38 may be chosen to substantially fill the chamber 36, or the foam 38 may leave one or more voids or clearances in the chamber 36. The foam 38 may be inserted into the chamber 36 prior to attachment of the flexible membrane 34 to the support wall 30, or the foam 38 may be injected into the chamber 36 via the port 32 after attachment of the flexible membrane 34 to the support wall 30. The support wall 30 bears the reaction force exerted by the foam 38. In the present embodiment, the foam 38 is generally cylindrically or disc shaped, although other embodiments could incorporate an elliptical or polygonal, rather than round, cross section.

A deodorizing filter 50 is installed on the other side of the support wall 30 to the flexible membrane 34 and the resilient device 38, for deodorizing flatus venting from the stoma 12. An inlet 50a of the filter 50 communicates with an annular space 52 inside the body waster collector 24 and surrounding the fluid filled seal 20. An outlet 50b of the filter 50 communicates with external atmosphere via one or more exhaust apertures 54 in the housing 18.

In use, the contact pressure exerted by the flexible membrane 34 on the stoma 12 is a combination of the force generated by compression of the resilient foam 38, and the pressure of fluid inside the chamber 36. The characteristics of the foam 38 may be chosen according to the desired pressure. In the present embodiment, where it is desired to present only a low contact pressure against the stoma 12, the foam 38 is generally soft. For example, the foam 38 has an Indention Force Deflection of approximately 30 lb./50 sq. in. force at 25% deflection. However an appropriate range for this value could be 10 lb./50 sq. in. to 45lb/50 sq. in. However, the characteristics of the foam 38 may be varied as desired. In the present embodiment, the foam 38 is an open cell foam, but a closed cell foam or a skinned foam may be used instead as desired. The Indentation Force Deflection measurement referred to above has unique units based on the method of testing. The reason is that the test is done with a presser foot with an area of 50 sq. In.

The end face 38a of the foam 38 adjacent to the flexible membrane 34 may be generally planar, or it may have a non-planar configuration. A non-planar configuration can (i) modify the local pressure response of the fluid filled seal 20, and/or (ii) modify the sealing properties of the fluid filled seal 20, and/or (iii) modify the fluid filled seal's 20 ability to conform to the shape of the stoma 12.

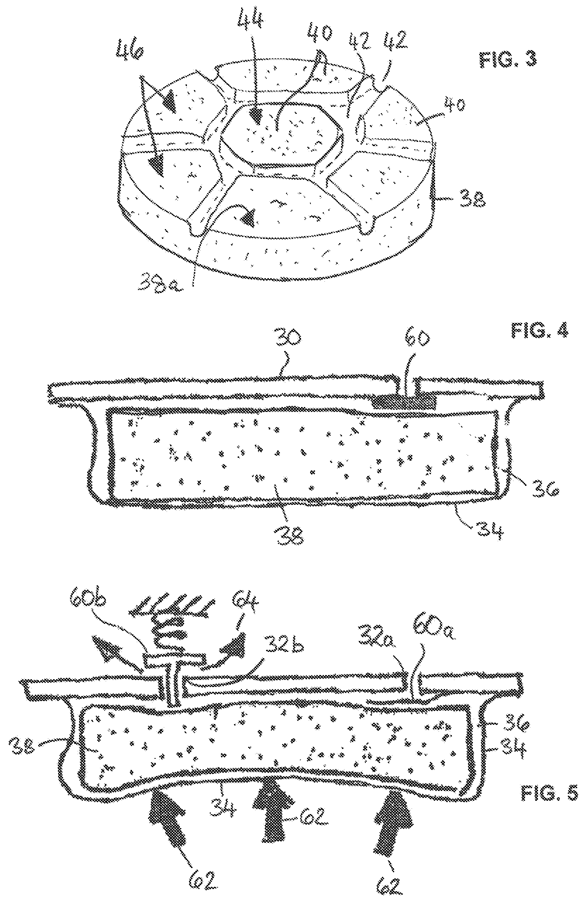

For example, the end face 38a of the foam 38 could be concave to match the typical protruding shape of the stoma 12. The concave surface may have a conical profile or a profile formed by a swept circular arc. In another form, the end face 38a of the foam may have a random, pseudo random, or regular texture, in order to enhance the sealing properties of the foam 38. The texture may provide regions, channels or paths of reduced local pressure concentration, to facilitate venting of flatus along corresponding paths on the opposite surface of the flexible membrane 34 at the interface with the stoma 12, without compromising the ability of the fluid filled seal 20 to prevent release of stool. In a further form illustrated in Fig. 3, the non-planar profile includes one or more ridges or grooves 42 between island or pad areas 40. The pad areas 40 may be shaped as one or more repeating polygons, radial arrays, or concentric circumferential rings or regions. The grooves 42 provide reduced pressure concentration along certain paths, and facilitate easier conformation of the block of foam 38, as described above. The non-planar profile may be generally divided into a central area 44 for contacting the stoma 12, and a surrounding area 46 for peristomal sealing. The illustrated non-planar configuration is merely an example, and other planar or non-planar configurations of the end face 38a may be used as desired for an intended application.

The fluid (air) pressure inside the chamber 36 is regulated by the port 32. In the first embodiment, the port 32 may be permanently open, allowing the chamber 36 to breath to external atmosphere. The contact pressure is generally equal to the pressure exerted by the foam 38, and the fluid pressure inside the chamber 36 is generally equal to atmospheric pressure outside the chamber 36, so that the fluid does not generate any additional contact pressure. Should the stoma 12 move inwardly or outwardly, fluid (air) is free to enter and/or exit the chamber 36 via the port 32 substantially unrestricted, so as to compensate for changes in the chamber volume. This means that the fluid pressure remains substantially constant at atmospheric pressure, not withstanding any short term pressure fluctuations that may occur as the stoma 12, moves, while air is drawn into, or expelled from, the chamber 36 to equalize the fluid pressure. For example, such pressure fluctuations may last no more than about 10 seconds. The first embodiment is thus configured to permit the flexible membrane 34 to follow changes in stoma 12 protrusion, while maintaining a controlled contact pressure determined substantially by the foam 38.

Fig. 4 depicts a second embodiment very similar to the first embodiment, except for the differences described below. Referring to Fig. 4, the port 32 is provided with a fluid flow control device 60 for controlling fluid flow through the port 32. The fluid flow control device 60 may be configured to control both inlet (admission) of fluid and exhaust (discharge) of fluid via the port 32, or to control the flow in only one direction. In the second embodiment shown in Fig. 4, the fluid filled seal 20 comprises a single port 32. In a third embodiment shown in Fig. 5 and described later, the fluid filled seal 20 comprises first and second ports 32a and 32b, and function of the fluid flow control device 60 is distributed amongst respective device 60a, 60b for the two ports 32a, 32b.

Referring to Fig. 4, the fluid flow control device 60 can control a damping action on the resilient force exerted by the foam 38, since the fluid flow control device 60 controls the degree to which fluid can enter or leave the chamber 36 in order for the fluid pressure inside the chamber 36 to equalize with respect to atmospheric pressure. The fluid flow control device 60 may provide the same fluid flow characteristics in both the inlet and outlet directions. Alternatively, the fluid flow control device 60 may provide different fluid inlet characteristics from outlet characteristics.

The fluid flow characteristics include one or both of: (i) resistance to flow of fluid through the port 32; and (ii) a valve action. The valve action may be defined by whether the valve is unidirectional or bidirectional, an opening pressure at which the valve opens to permit flow, and whether the valve remains permanently open once opened for the first time or whether the valve re-closes.

For example, the fluid flow control device 60 may restrict the inlet flow rate of fluid by means of a flow constriction. In one form, restricting the flow into the fluid filled seal 20 would cause the fluid filled seal 20 to inflate more slowly under the expansive influence of the foam 38 contained within the fluid filled seal 20. This would allow the fluid filled seal 20 to respond slowly to retraction of the stoma 12. For example, whereas an unrestricted inlet port 32 might allow the seal to inflate fully from a fully compressed state in less than 10 seconds, a fluid flow control device 60 could change the inflation time to 30 minutes or more.

In another form, the fluid flow control device 60 is configured to permit inlet of fluid into the chamber 36 more easily than permitting exhaust of fluid from the chamber 36. The fluid flow control device 60 comprises a valve (not shown) configured (i) to open, in the inlet direction, with a relatively small pressure differential across the valve (for example, an opening pressure of not more than 9mm Hg. and/or (ii) to provide a relatively small resistance to flow of fluid in the inlet direction (for example, airflow of 50cc/min or greater). This enables the flexible membrane 34 to conform to movement of the stoma 12 rapidly when the stoma 12 moves inwardly with respect to the skin surface. Fluid can be sucked into the chamber 36 rapidly, such that expansion of the foam 38 and the flexible membrane 34 is substantially undamped. In contrast, the fluid flow control device 60 is configured to open, in the outlet direction, with a higher pressure differential across the valve than in the inlet direction (for example, an opening pressure of about 15mm Hg and/or to provide a relatively higher resistance to flow of fluid in the outlet direction than in the inlet direction (for example 3cc/min). Such characteristics damp changes in the fluid filled seal 20 when the stoma 12 moves outwardly with respect to the skin surface, yet manage the fluid pressure to avoid prolonged increase in contact pressure. This enables the fluid filled seal 20 to handle a brief challenge from the stoma 12 under the pressure of stool. Should the stoma 12 move outwardly under pressure of stool, the fluid trapped in the chamber 36 results in an increased fluid pressure to increase the contact pressure against the stoma 12. The increased contact pressure can aid blocking the release of stool temporarily. However, the fluid flow control device 60 does permit fluid to be discharged from the chamber 36 with a damped response, such that the increased fluid pressure is relieved over time or within certain limits, to ensure that a higher than desired contact pressure is not maintained for a prolonged period of time, and so there is little risk of tissue damage as a result of reduce blood perfusion.

In the second embodiment, a fluid flow control device 60 is illustrated, and the fluid flow control device 60 performs both an inlet and an outlet function. In the third embodiment illustrated in Fig. 5, the fluid filled seal 20 comprises first and second ports 32a, 32b, each with a respective flow control, first flow control 60a, second flow control 60b. One or both of the flow controls 60a, 60b may allow bidirectional flow, or one or both of the flow controls 60a, 60b may allow flow in only one direction. In the illustrated form, the first port 32a serves as an inlet port, and the first flow control 60a comprises an inlet valve configured for controlling the admission of fluid into the chamber 36 (and blocking discharge of fluid through the port 32a). The second port 32b serves as an exhaust port, and the second flow control 60b comprises an exhaust valve configured for controlling the discharge of fluid from the chamber 36 (and blocking inlet of fluid through the port 32b). For example, when the stoma 12 moves in an outward direction as depicted by arrows 62, the second flow control 60b controls discharge of fluid (illustrated by arrows 64) to provide the fluid filled seal 20 with a damped response, as explained above.

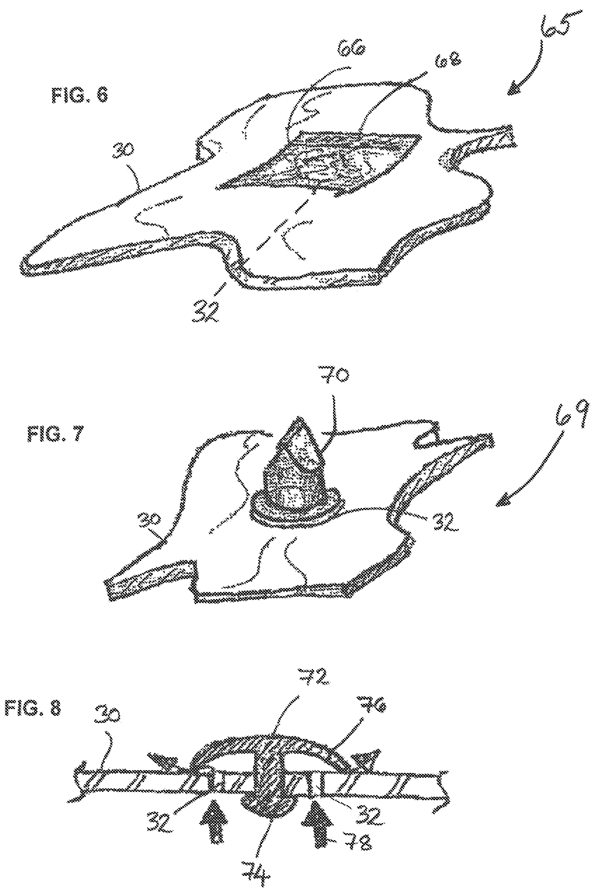

The fluid flow control device 60, and the flow controls 60a, 60b may be of any suitable type(s), and examples are shown in Figs. 6-15.

Fig. 6 illustrates a flap valve 65 generally comprising a seal flap 66 attached to the support wall 30 over the port 32 by one or more attachment regions 68. When the fluid pressure on the opposite side of the support wall 30 exceeds the pressure on the flap-side of the support wall 30 by a valve threshold, the flap 66 lifts from the support wall 30 to allow fluid to pass through the port 32. When the pressure on the flap-side of the support wall 30 is greater, the fluid bears on the flap 66 urging it into sealing contact with the support wall 30 to prevent fluid flow through the port 32.

Fig. 7 illustrates a duck-bill type valve 69, that operates in a similar way, except that the duck-bill type valve 69 comprises confronting lips.

Fig. 8 illustrates an umbrella valve 72 comprising a skirt 76 supported by a central stem 74 anchored with respect to the support wall 30. When the fluid pressure on the opposite side of the support wall 30 exceeds the pressure on the skirt-side of the support wall 30 by a valve threshold, the skirt 76 lifts from the support wall 30 to allow passage of fluid through the port apertures 32, as illustrated by arrows 78. When the fluid pressure on the skirt-side of the support wall 30 is greater than that on the opposite side of the support wall 30, the fluid bears on the skirt 76, to press the skirt 76 into sealing engagement with the support wall 30, and thereby block fluid flow through the port apertures 32.

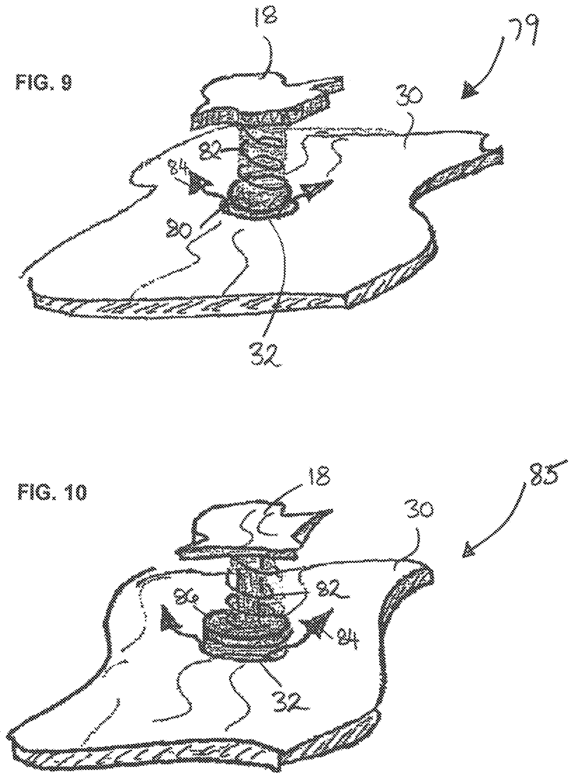

Fig. 9 illustrates a ball valve 79 comprising a ball 80 that is pressed into sealing engagement with a seat of the port 32 by means of a spring 82. When the fluid pressure on the opposite side of the support wall 30 exceeds a valve threshold set by the spring force, the pressure lifts the ball 80 slightly out of sealing engagement with the seat at the port 32, and allows fluid to pass through the port 32 as illustrated by arrows 84. When the fluid pressure on the opposite side of the support wall 30 drops below the valve threshold, the spring 82 urges the ball 80 into sealing engagement to close the port 32.

Fig. 10 illustrates a poppet valve 85 similar to the ball valve of Fig. 9, except that the valve member is a poppet head 86 instead of a ball 80.

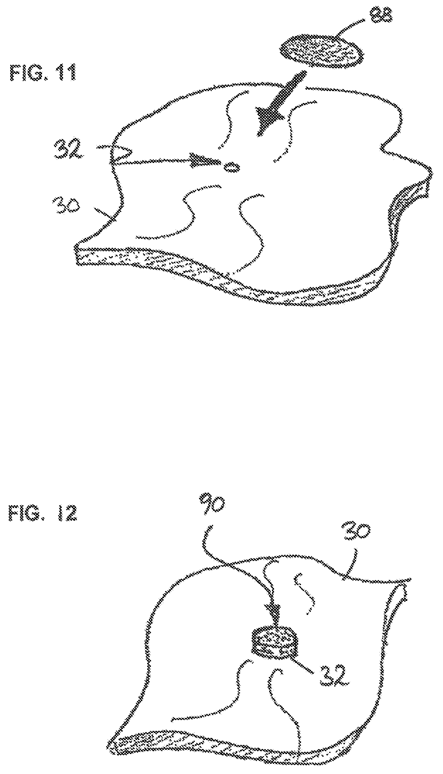

Fig. 11 illustrates a flow-restrictor for the port 32, in the form of a porous membrane 88, for example, a microporous membrane. The porous membrane 88 is adhered to the surface of the support wall 30 around the port 32, in order to control the flow rate of fluid through the port 32. A flow restrictor may be used in combination with a valve, or the port 32 may be unvalved and left open except for the flow-restrictor. For example, in the embodiment of Fig. 5, the exhaust valve 60b may be replaced by the flow-restrictor. This would provide a permanently open port 32b, but having a substantially restricted flow rate. Fluid may still be admitted rapidly into the chamber 36 by means of the inlet valve 60a, but gas discharged from the chamber 36 has to pass through the flow restrictor, thereby providing the damped response as described for the third embodiment, but without the need for the exhaust valve 60b.

Alternatively, the fluid control device 60 in Fig. 5 may be fitted with a porous membrane 88 to allow flow at a low rate even when the fluid control device 60 is closed.

Fig. 12 illustrates an alternative flow-restrictor in the form of a porous plug 90 disposed in the port 32. The plug 90 is made, for example, of microporous material.

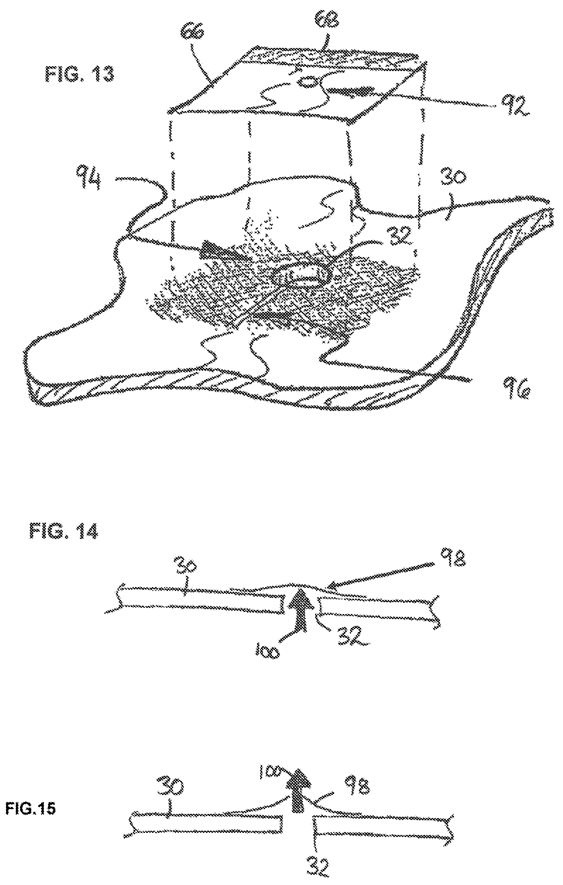

Fig. 13 illustrates a fluid control device 60 modified to have a "leaky" or imperfect seal characteristic. The fluid flow control device 60 is based on the flap valve 65 shown in Fig. 6, but the same principles may be applied to any of the other valves. The imperfect seal is provided by one or more of: (i) at least a portion 94 of the valve seat surface being interrupted or having a textured surface; (ii) a precise groove or scratch 96 extending at least partly across the valve seat surface; and/or (iii) a small hole in the seal flap 66 itself that allows fluid to leak therethrough. Although all three imperfect seal features are shown in combination in Fig. 13, it will be appreciated that any two or one of the features may be implemented as desired.

Figs. 14 and 15 illustrate a safety valve for permanently and rapidly opening the port 32 should the fluid pressure in the chamber exceed a threshold. The safety valve comprises a rupturable membrane 98 extending over or across the port 32. When the pressure (indicated by arrow 100) exceeds a rupture pressure of the membrane 98, the membrane 98 ruptures to permanently open the port 32, and allow escape of the fluid.

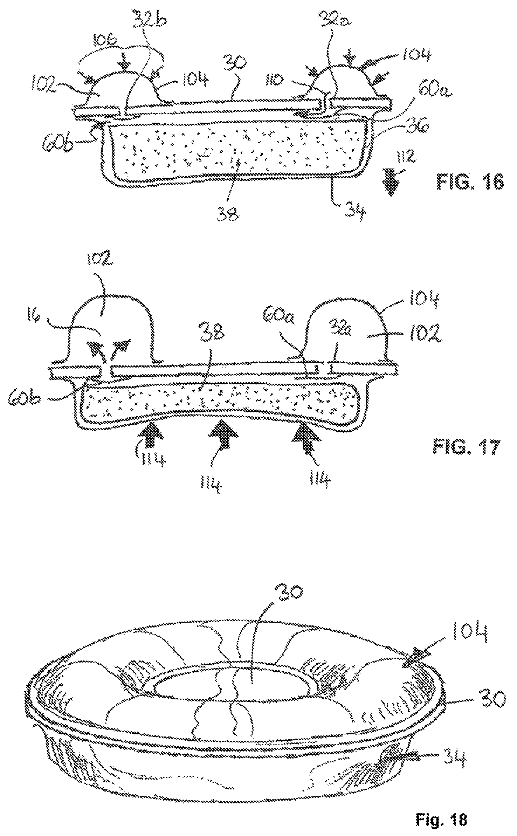

In the preceding embodiments, the fluid filled seal 20 comprises a single chamber 36 that communicates via the port(s) 32 with external atmosphere. The inflation fluid used in the preceding embodiments is air. Figs. 16-18 illustrate a fourth embodiment comprising a second chamber 102 that acts as a reservoir for the fluid, the second chamber 102 communicating with the first chamber 36 via at least one port 32. The first and second chambers 36, 102 form, at least in use, a closed system. The fluid contained by the first and second chambers 36, 102 may be air or another gas, or it may be a liquid, such as saline, or a flowable gel. The second chamber 102 has different properties from the first chamber 36, such as a different elasticity. Fluid may be transferable freely from one chamber to the other to compensate for expansion and contraction of the first chamber 36. This would create a responsive system that reacts to forces applied to the flexible membrane 34 by transferring fluid between the two chambers 36, 102. The volume and pressure characteristics determine the change in volume and pressure of the first chamber 36 as is challenged by an external force. Therefore, the properties of the second chamber 102 could be selected or optimized to maintain a controlled fluid pressure in the first chamber 36 acting on the flexible membrane 34 under changing conditions.

In the form illustrated in Figs. 16-18, fluid transfer between the first and second chambers 36, 102 is controlled by one of more flow controls 60a, 60b. The flow controls 60a, 60b may be similar to any of the valves and/or flow restrictors described above. In a similar manner to that described previously, fluid flow from the second chamber 102 to the first chamber 36 could be relatively unrestricted (undamped), whereas return fluid flow from the first chamber 36 into the second chamber 102 may be controlled by a second flow control 60b in the form of a pressure relief valve and/or a flow restrictor (damped response).

It will be appreciated that the foregoing description is illustrative of preferred forms of the invention, and that many modifications, may be made without departing from the scope of the invention as claimed.

## Claims

1. A controlled evacuation ostomy appliance (10) comprising a fluid filled seal (20) for sealing against the human body, the seal (20) having:
a fluid chamber (36, 102) including a fluid impermeable membrane (34) that forms a movable wall of the fluid chamber (36, 102);
one or more ports (32, 32a, 32b) communicating with the chamber (36, 102);
**characterized in that** the seal (20) further comprises a resilient device (38) disposed within the fluid chamber (36, 102).

2. The controlled evacuation ostomy appliance (10) according to claim 1, wherein the resilient device (38) is configured to urge the membrane (34) in a direction (i) for forming a seal in use and/or (ii) for expanding the chamber (36, 102).

3. The controlled evacuation ostomy appliance (10) according to claim 1, wherein the resilient device (38) comprises foam (38).

4. The controlled evacuation ostomy appliance (10) according to claim 3, wherein the foam (38) has a shape selected from: a generally cylindrical block; a block with an elliptical cross section; a block with a polygonal cross section.

5. The controlled evacuation ostomy appliance (10) according to claim 3, wherein the foam (38) has a generally non-planar surface on a face that faces towards or away from the membrane (34), the non-planar surface providing local variation in the pressure exerted by the foam on the membrane (34).

6. The controlled evacuation ostomy appliance (10) according to claim 1, wherein at least one port (32, 32a, 32b) permits fluid to enter and to leave the fluid chamber (36), at least when a pressure threshold is reached.

7. The controlled evacuation ostomy appliance (10) according to claim 6, wherein at least one port (32, 32a, 32b) defines a fluid flow characteristic that is the same in an inlet direction and an outlet direction.

8. The controlled evacuation ostomy appliance (10) according to claim 7, wherein at least one port (32) defines a fluid flow characteristic that is different in an inlet direction than in an outlet direction, and wherein the fluid flow characteristic is at least one selected from:
(i) permits fluid to enter the chamber (36, 102) more easily than permitting fluid to leave the chamber (36, 102);
(ii) is a first resistance to flow of fluid into the chamber (36, 102), and second resistance to flow of fluid out of the chamber (36, 102), the second resistance being different from the first resistance;
(iii) is a valve (60a, 65, 69, 72, 85) opening at a first pressure differential for inlet of fluid into the chamber (36, 102), and valve (60b, 65, 69, 72, 85) opening at a second pressure differential for exhaust of fluid from the chamber (36, 102), the magnitude of first pressure differential being different from the magnitude of the second pressure differential.

9. The controlled evacuation ostomy appliance (10) according to claim 1, wherein at least one port (32, 32a, 32b) comprises at least one fluid flow control device (60) for controlling the flow of fluid through the port (32, 32a, 32b).

10. The controlled evacuation ostomy appliance (10) according to claim 9, wherein the fluid flow control device (60) comprises at least one selected from:
(i) a valve (60a, 60b, 65, 69, 72, 85);
(ii) a microporous membrane (88);
(iii) a microporous plug (90);
(iv) a check valve (60a) configured to obstruct flow in one direction and to permit flow in an opposite direction;
(v) a flow resistance for restricting the flow through the port (32, 32a, 32b);
(vi) a valve (60a, 60b, 65, 69, 72, 85) in combination with the flow resistance (v); and
(vii) a fluid flow control device that (60) is configured to open permanently when a pressure threshold is reached.

11. The controlled evacuation ostomy appliance (10) according to claim 9, wherein the fluid control device (60) comprises a flow resistance for restricting flow through the port (32, 32a, 32b), and a valve (60a, 60b, 65, 69, 72, 85) in combination with the flow resistance, wherein valve (60a, 60b, 65, 69, 72, 85) is configured to permit fluid flow with a smaller resistance in one direction than the other.

12. The controlled evacuation ostomy appliance (10) according to claim 1, further comprising a second chamber (102) coupled to the at least one port (32, 32a, 32b).

13. The controlled evacuation ostomy appliance (10) according to claim 12, wherein the first and second chambers (36, 102) define, in use, a closed volume of fluid.

14. The controlled evacuation ostomy appliance (10) according to claim 1, wherein the fluid is selected from: a gas; a liquid; a gel.

## Patentansprüche

1. Ostomievorrichtung zur kontrollierten Entleerung (10) umfassend eine fluidgefüllte Abdichtung (20) zum Abdichten gegenüber dem menschlichen Körper, wobei die Abdichtung (20) Folgendes aufweist:
eine Fluidkammer (36,102) umfassend eine fluidundurchlässige Membran (34), die eine bewegliche Wand der Fluidkammer (36,102) bildet;
einen oder mehrere Anschlüsse (32,32a, 32b), die mit der Kammer (36, 102) in Verbindung stehen;
**dadurch gekennzeichnet, dass** die Abdichtung (20) weiter eine federnde Vorrichtung (38) umfasst, die innerhalb der Fluidkammer (36,102) angeordnet ist.

2. Ostomievorrichtung zur kontrollierten Entleerung (10) nach Anspruch 1, wobei die federnde Vorrichtung (38) so konfiguriert ist, dass sie die Membran (34) in eine Richtung (i) drängt, um eine Abdichtung bei der Anwendung zu bilden und/oder (ii) die Kammer auszudehnen (36,102).

3. Ostomievorrichtung zur kontrollierten Entleerung (10) nach Anspruch 1, wobei die federnde Vorrichtung (38) Schaumstoff (38) umfasst.

4. Ostomievorrichtung zur kontrollierten Entleerung (10) nach Anspruch 3, wobei der Schaumstoff (38) eine Form aufweist, die ausgewählt wurde aus: einem im Allgemeinen zylindrischen Block; einem Block mit einem elliptischen Querschnitt; einem Block mit einem polygonalen Querschnitt.

5. Ostomievorrichtung zur kontrollierten Entleerung (10) nach Anspruch 3, wobei der Schaumstoff (38) eine im Allgemeinen nichtplanare Oberfläche an einer Seite aufweist, die auf die Membran (34) hin oder von dieser weg zeigt, wobei die nichtplanare Oberfläche eine lokale Veränderung des Druckes, der durch den Schaum auf die Membran (34) ausgeübt wird, erzeugt.

6. Ostomievorrichtung zur kontrollierten Entleerung (10) nach Anspruch 1, wobei mindestens ein Anschluss (32, 32a, 32b) es erlaubt, dass Fluid in die Fluidkammer (36) eintritt und diese verlässt, zumindest wenn eine Druckschwelle erreicht wird.

7. Ostomievorrichtung zur kontrollierten Entleerung (10) nach Anspruch 6, wobei mindestens ein Anschluss (32, 32a, 32b) ein Strömungsverhalten des Fluids definiert, das in einer Einlassrichtung und in einer Auslassrichtung gleich ist.

8. Ostomievoruchtung zur kontrollierten Entleerung (10) nach Anspruch 7, wobei mindestens ein Anschluss (32) ein Strömungsverhalten des Fluids definiert, das sich in einer Auslassrichtung von demjenigen in einer Einlassrichtung unterscheidet, und wobei das Strömungsverhalten des Fluids mindestens eines der Folgenden ist:
(i) ermöglicht es dem Fluid, leichter in die Kammer (36,102) einzutreten, als es dem Fluid ermöglicht wird, die Kammer (36,102) zu verlassen;
(ii) ist ein erster Widerstand zur Fluidströmung in die Kammer (36,102) hinein und ein zweiter Widerstand zur Fluidströmung aus der Kammer (36,102) heraus, wobei sich der zweite Widerstand vom ersten Widerstand unterscheidet;
(iii) ist ein Ventil (60a, 65, 69, 72, 85), das sich bei einem ersten Differenzdruck zum Einlassen von Fluid in die Kammer (36,102) öffnet, und ein Ventil (60b, 65, 69, 72, 85), das sich bei einem zweiten Differenzdruck zum Auslassen von Fluid aus der Kammer (36,102) öffnet, wobei sich die Größe des ersten Differenzdrucks von der Größe des zweiten Differenzdrucks unterscheidet.

9. Ostomievoruchtung zur kontrollierten Entleerung (10) nach Anspruch 1, wobei mindestens ein Anschluss (32, 32a, 32b) mindestens eine Fluidströmungssteuerungsvorrichtung (60) umfasst, um die Fluidströmung durch den Anschluss (32, 32a, 32b) zu steuern.

10. Ostomievorrichtung zur kontrollierten Entleerung (10) nach Anspruch 9, wobei die Fluidströmungssteuerungsvorrichtung (60) mindestens eines der folgenden Elemente umfasst:
(i) ein Ventil (60a, 60b, 65, 69, 72, 85);
(ii) eine mikroporöse Membran (88);
(iii) einen mikroporösen Stopfen (90);
(iv) ein Rückschlagventil (60a), das konfiguriert ist, um die Strömung in eine Richtung zu blockieren und um die Strömung in eine entgegengesetzte Richtung zu erlauben;
(v) einen Strömungswiderstand, um die Strömung durch den Anschluss (32, 32a, 32b) zu drosseln;
(vi) ein Ventil (60a, 60b, 65, 69, 72, 85) in Kombination mit dem Strömungswiderstand (v); und
(vii) eine Fluidströmungssteuerungsvorrichtung (60), die so konfiguriert ist, dass sie sich permanent öffnet, wenn eine Druckschwelle erreicht ist.

11. Ostomievorrichtung zur kontrollierten Entleerung (10) nach Anspruch 9, wobei die Fluidsteuerungsvorrichtung (60) einen Strömungswiderstand, um die Strömung durch den Anschluss (32, 32a, 32b) zu drosseln, und ein Ventil (60a, 60b, 65, 69,72,85) in Kombination mit dem Strömungswiderstand umfasst, wobei das Ventil (60a, 60b, 65, 69,72, 85) so konfiguriert ist, dass es eine Fluidströmung mit einem geringeren Widerstand in eine Richtung als in die andere Richtung erlaubt.

12. Ostomievorrichtung zur kontrollierten Entleerung (10) nach Anspruch 1 weiter umfassend eine zweite Kammer (102), die mit mindestens einem Anschluss (32, 32a, 32b) verbunden ist.

13. Ostomievorrichtung zur kontrollierten Entleerung (10) nach Anspruch 12, wobei die erste und die zweite Kammer (36, 102) bei der Verwendung ein geschlossenes Fluidvolumen definieren.

14. Ostomievorrichtung zur kontrollierten Entleerung (10) nach Anspruch 1, wobei das Fluid ausgewählt wird aus: einem Gas; einer Flüssigkeit; einem Gel.

## Revendications

1. Appareil d'évacuation commandée de stomie (10) comprenant une capsule remplie de fluide (20) destinée à fermer hermétiquement le corps humain, la capsule (20) comportant :
une chambre de fluide (36, 102) comprenant une membrane imperméable au fluide (34) qui forme une paroi mobile de la chambre de fluide (36, 102) ;
un ou plusieurs orifices (32, 32a, 32b) communiquant avec la chambre (36, 102);
**caractérisé en ce que** la capsule (20) comprend en outre un dispositif souple (38) disposé à l'intérieur de la chambre de fluide (36, 102).

2. Appareil d'évacuation commandée de stomie (10) selon la revendication 1, dans lequel, en utilisation, le dispositif souple (38) est conçu pour pousser la membrane (34) dans un sens (i) permettant d'assurer une fermeture hermétique et/ou (ii) permettant de dilater la chambre (36, 102).

3. Appareil d'évacuation commandée de stomie (10) selon la revendication 1, dans lequel le dispositif souple (38) comprend de la mousse (38).

4. Appareil d'évacuation commandée de stomie (10) selon la revendication 3, dans lequel la mousse (38) a une forme choisie parmi plusieurs formes constituées : d'un bloc globalement cylindrique ; d'un bloc à section transversale elliptique ; d'un bloc à section transversale polygonale.

5. Appareil d'évacuation commandée de stomie (10) selon la revendication 3, dans lequel la mousse (38) comporte une surface globalement non planaire située sur une face qui est orientée vers la membrane (34), ou à l'opposé de cette dernière, la surface non planaire fournissant une variation locale de la pression exercée par la mousse sur la membrane (34).

6. Appareil d'évacuation commandée de stomie (10) selon la revendication 1, dans lequel au moins un orifice (32, 32a, 32b) permet l'entrée d'un fluide dans la chambre de fluide (36) et la sortie dudit fluide de cette dernière, au moins lorsqu'un seuil de pression a été atteint.

7. Appareil d'évacuation commandée de stomie (10) selon la revendication 6, dans lequel au moins un orifice (32, 32a, 32b) définit une caractéristique d'écoulement de fluide qui est la même dans un sens d'entrée et dans un sens de sortie.

8. Appareil d'évacuation commandée de stomie (10) selon la revendication 7, dans lequel au moins un orifice (32) définit une caractéristique d'écoulement de fluide dans un sens d'entrée, différente de celle d'un sens de sortie, et dans lequel la caractéristique d'écoulement de fluide est au moins choisie parmi les actions suivantes :
(i) permettre une entrée de fluide dans la chambre (36, 102) plus facile qu'une sortie de fluide de la chambre (36, 102) ;
(ii) établir une première résistance à l'écoulement de fluide en entrée dans la chambre (36, 102), et une seconde résistance à l'écoulement de fluide en sortie de la chambre (36, 102), la seconde résistante étant différente de la première résistance ;
(iii) ouvrir une soupape (60a, 65, 69, 72, 85) à un premier différentiel de pression pour l'entrée de fluide dans la chambre (36, 102), et ouvrir une soupape (60b, 65, 69, 72, 85) à un second différentiel de pression pour la sortie de fluide de la chambre (36, 102), l'importance du premier différentiel de pression étant différente de l'importance du second différentiel de pression.

9. Appareil d'évacuation commandée de stomie (10) selon la revendication 1, dans lequel au moins un orifice (32, 32a, 32b) comprend au moins un dispositif de commande d'écoulement de fluide (60) destiné à commander l'écoulement de fluide à travers l'orifice (32, 32a, 32b).

10. Appareil d'évacuation commandée de stomie (10) selon la revendication 9, dans lequel le dispositif de commande d'écoulement de fluide (60) comprend au moins l'un :
(i) d'une soupape (60a, 60b, 65, 69, 72, 85) ;
(ii) d'une membrane microporeuse (88) ;
(iii) d'un obturateur microporeux (90) ;
(iv) d'une soupape anti-retour (60a) conçue pour obstruer l'écoulement dans un sens et permettre l'écoulement dans un sens contraire ;
(v) d'une résistance à l'écoulement destinée à limiter l'écoulement à travers l'orifice (32, 32a, 32b) ;
(vi) d'une soupape (60a, 60b, 65, 69, 72, 85) combinée à la résistance à l'écoulement (v) ; et
(vii) d'un dispositif de commande d'écoulement de fluide (60) conçu pour s'ouvrir de façon permanente lorsque le seuil de pression a été atteint.

11. Appareil d'évacuation commandée de stomie (10) selon la revendication 9, dans lequel le dispositif de commande de fluide (60) comprend une résistance à l'écoulement destinée à limiter l'écoulement à travers l'orifice (32, 32a, 32b), et une soupape (60a, 60b, 65, 69, 72, 85) combinée à la résistance à l'écoulement, dans lequel la soupape (60a, 60b, 65, 69, 72, 85) est conçue pour permettre un écoulement de fluide ayant une résistance plus faible dans un sensque dans l'autre.

12. Appareil d'évacuation commandée de stomie (10) selon la revendication 1, comprenant en outre une seconde chambre (102) couplée audit orifice (32, 32a, 32b).

13. Appareil d'évacuation commandée de stomie (10) selon la revendication 12, dans lequel les première et seconde chambres (36, 102) définissent, en utilisation, un volume fermé de fluide.

14. Appareil d'évacuation commandée de stomie (10) selon la revendication 1, dans lequel le fluide est choisi parmi : un gaz ; un liquide ; un gel.
